(19) Europäisches Patentamt
European Patent Office
Office européen des brevets

(11) Numéro de publication : 0 380 409 B1

(12) # FASCICULE DE BREVET EUROPEEN

(45) Date de publication du fascicule du brevet : **01.06.94 Bulletin 94/22**

(51) Int. Cl.$^5$ : **C07C 315/00,** C07C 319/02, B01F 17/00, C07D 407/12

(21) Numéro de dépôt : **90400196.3**

(22) Date de dépôt : **24.01.90**

(54) **Procédé de préparation de tensio-actifs non-ioniques à partir de l'isopropylidène-1,2 époxypropyl-3 glycérol et de composés hydroxylés, nouveaux tensio-actifs non ioniques et leur utilisation.**

(30) Priorité : **26.01.89 FR 8900961**

(43) Date de publication de la demande : **01.08.90 Bulletin 90/31**

(45) Mention de la délivrance du brevet : **01.06.94 Bulletin 94/22**

(84) Etats contractants désignés : **AT BE CH DE DK ES FR GB IT LI NL SE**

(56) Documents cités :
**EP-A- 0 025 307**
**EP-A- 0 042 505**
**EP-A- 0 071 019**
**CH-A- 474 463**
**CH-A- 490 302**
**CH-A- 518 716**
**DE-A- 1 961 731**

(56) Documents cités :
**FR-A- 1 484 723**
**FR-A- 1 561 585**
**FR-A- 2 482 128**
**FR-A- 2 593 509**
**GB-A- 2 060 665**
**GB-A- 2 144 122**
**GB-A- 2 144 438**

(73) Titulaire : **L'OREAL**
**14, Rue Royale**
**F-75008 Paris (FR)**

(72) Inventeur : **Sebag, Henri**
**26, rue Erlanger**
**F-75016 Paris (FR)**

(74) Mandataire : **Casalonga, Axel et al**
**BUREAU D.A. CASALONGA - JOSSE**
**Morassistrasse 8**
**D-80469 München (DE)**

EP 0 380 409 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un nouveau procédé de préparation de tensio-actifs non ioniques, les compositions les contenant ainsi que l'utilisation de ces compositions dans l'industrie des cosmétiques, des produits pharmaceutiques et des produits textiles.

Le nouveau procédé consiste dans une première étape, à condenser sur des composés comportant au moins un groupement OH l'isopropylidène-1,2 époxypropyl-3 glycérol (IPEG) de formule (I)

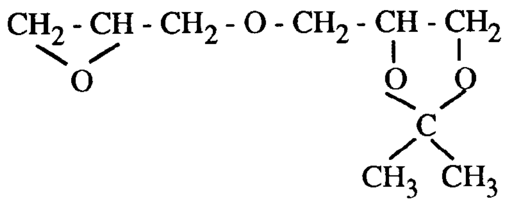

(I)

puis dans une deuxième étape à hydrolyser les produits obtenus, conduisant à des produits non-ioniques polyhydroxylés.

FR-A-1 484 723 décrit des composés tensio-actifs de formule

$$R\text{-}O\text{-}[C_2H_3O(CH_2OCH_2\text{-}CHOH\text{-}CH_2OH)]_n\,H \qquad (F_1)$$

Ces produits de formule ($F_1$) sont obtenus par réaction de l'allylglycidyléther avec un alcool gras, suivie d'une hydroxylation des doubles liaisons avec de l'eau oxygénée à 130 volumes (39 %), en présence d'acide formique à 98 %, pendant 24 à 48 heures, à 40°C. L'utilisation d'eau oxygénée et d'acide concentrés présente des risques, tant du point de vue de la sécurité que du point de vue de la formation de produits secondaires non désirés.

EP-A-0071019 décrit la préparation d'un 2,3-dialkoxy propyl glycéryl éther en 4 étapes, comprenant (i) la préparation d'un glycidyl éther à partir d'un alcool (ii) la réaction du glycidyl éther avec un 1,3-dioxolane, (iii) la transformation du 1,3-dioxolane en dialkyl éther de dioxolane (iv) l'hydrolyse du dialkyl éther de dioxolane par chauffage à 50-100°C.

Ce procédé enseigne, la préparation d'un composé de monoaddition. Il ne prévoit pas de composés de polyaddition.

DE-A-1 961 731 décrit un procédé de préparation d'éthers et de polyéthers polyhydroxylés, en deux étapes. Dans une première étape on fait réagir le tertio-butyl glycidyl éther avec un composé contenant au moins un hydrogène réactif. La deuxième étape consiste à hydrolyser les groupements tertio-butoxy par chauffage, en présence d'un catalyseur, à une température de 80 à 110°C et de préférence à une température de 90 à 100°C.

FR-A-2 482 128 décrit la préparation de tensio-actifs polyethers polyhydroxylés de formule

$$R\text{-}O\text{-}[(C_2H_3)(OZ)]\,R_1$$

ou Z peut désigner

$$-\,[(C_2H_3O)(CH_2OH)]_n\text{—}H$$

par un procédé en trois étapes comprenant (i) la réaction d'un alcool ROH avec un composé époxy

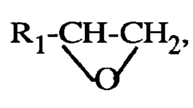

(ii) la polyaddition du tertio-butyl glycidol éther, (iii) l'hydrolyse des dérivés polytertiobutoxy en présence d'un acide fort à 80°-110°C.

CH-A- 474 463 enseigne des tensio-actifs non-ioniques comportant une seule chaîne lipophile pour deux groupes hydrophiles. Ces composés peuvent être préparés par polyaddition d'épihalohydrine sur un alphadiol suivi d'une hydroxylation par un sel alcalin carboxylique à une température entre 130 et 230°C.

FR-A- 2 593 509 enseigne des tensio-actifs non ioniques de formule

$$R\text{-}CH_2\text{-}CH_2\text{-}\underset{\displaystyle R}{CH}\text{-}CH_2\text{-}O\ [C_2\,H_3\,O\,[(CH_2\,OH)]_n\ H$$

où R désigne le radical décyle ou dodécyle.

Ces produits peuvent être préparés d'une manière similaire à celle décrite par CH-A- 474 463.

CH-A- 490 302 décrit des tensioactifs non ioniques de formule

$$RO\,[C_2H_3\ (R')]_m\ \ [C_2\,H_3\ (X)]_n\text{—}H \qquad (I)$$

où R est un radical aliphatique en $C_{12}$-$C_{20}$

R′ est méthyle ou éthylène

X désigne un radical hydroxyméthyle ou un groupe polyhydroxypolyéther.

Ces composés sont préparés par polyaddition sur un alcool gras ROH d'abord d'un époxyde puis d'une épihalohydrine. Les composés intermédiaires ainsi obtenus sont hydroxylés à une température comprise entre 150° et 200°C.

Les différents procédés ci-dessus de l'art antérieur comportent tous la préparation, en une ou plusieurs étapes, d'un composé intermédiaire dont l'hydrolyse ou l'hydroxylation conduit au composé tensio-actif non ionique recherché. L'hydrolyse ou l'hydroxylation sont toujours effectués par chauffage à des températures de 50 à 200°C. Un chauffage prolongé présente divers inconvénients surtout lorsqu'il est réalisé à l'échelle industrielle. De plus le chauffage peut être néfaste pour la préparation de produits thermo-sensibles.

Le procédé selon la présente invention résoud ce problème. Il permet de réaliser la réaction de polyaddition de l'IPEG de la première étape, en présence d'un catalyseur acide de Lewis, généralement à une température comprise entre 20 et 80°C et surtout il permet de réaliser la réaction d'hydrolyse de la deuxième étape, à la température ambiante.

Le procédé de la présente invention consiste à préparer des produits tensioactifs non-ioniques de formule (IV), à partir de l'isopropylidène- 1,2 époxypropyl-3 glycérol (IPEG) en deux étapes.

Dans la première étape on fait réagir un alcool de formule (II) comportant une ou plusieurs fonctions hydroxyles sur l'IPEG de formule I selon le schéma réactionnel suivant

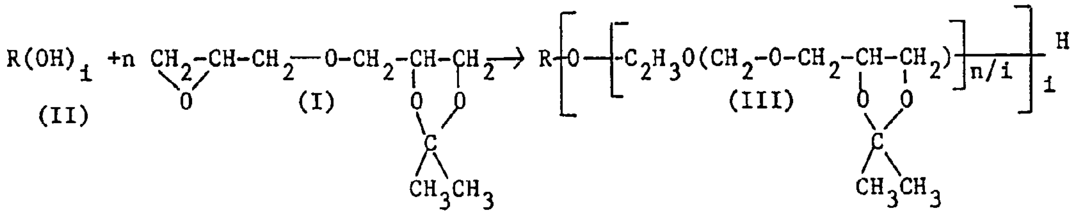

Dans la deuxième étape on hydrolyse le composé intermédiaire de formule (III), à la température ambiante, en milieu hydro-alcoolique, en présence d'un catalyseur acide, en obtenant les composés hydroxylés de formule (IV), selon le schéma réactionnel

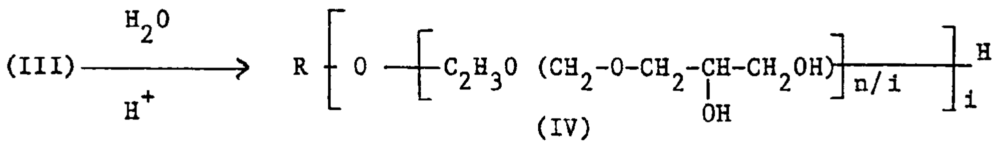

(IV)

Dans la formule (IV) ci-dessus le groupement

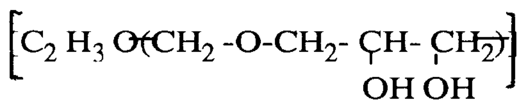

désigne l'un ou l'autre groupement suivant, selon le sens d'ouverture de l'époxyde

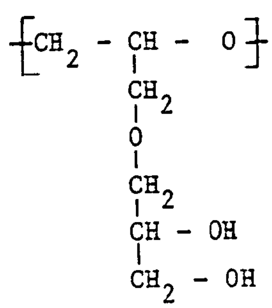 (V) ou 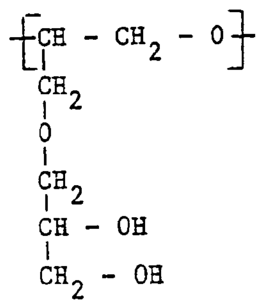 (VI)

Dans les formules ci-dessus

i désigne 1,2 ou 3

n est un nombre entier ou décimal de 1,5 à 10 et

désigne le nombre statistique moyen de moles de composé (I) mises en oeuvre par mole de composé (II), le nombre réel des motifs hydrophiles par chaîne grasse pouvant être inférieur, égal ou supérieur au nombre n.

R désigne le radical

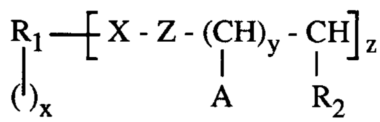 (VII)

dans laquelle

$R_1$ désigne un radical mono ou divalent, aliphatique, cycloaliphatique ou alcoylarylique en $C_8$-$C_{36}$,

$R_2$ désigne H, un radical alcoyle, alcoxyméthyle, alcényle ou alcényloxyméthyle en $C_8$ à $C_{23}$,

X désigne O, S, S(0), COO, CONH ou $CH_2$,

Z désigne $CH_2$ ou $(CH_2\text{-}CH_2\text{-}O)_m$

m est un nombre entier ou décimal de 1 à 20,

A désigne H ou une liaison covalente,

x, y et z désignent indépendamment l'une de l'autre 0 ou 1

sous réserve que lorsque Z désigne $(CH_2\text{-}CH_20)_m$ alors y est égal à 1 et A = $R_2$= H;

Le radical

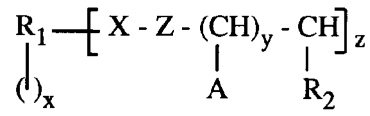

(VII)

englobe les deux radicaux ci-après

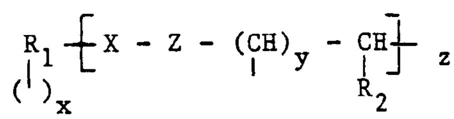

(VIII),

et

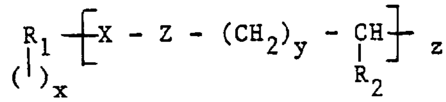

(IX)

Dans la formule $A(OH)_i$ $i = x + y + z$.

Quand ce réactif $R(OH)_i$ désigne un polyol ($i > 1$), chaque groupe hydroxyle peut réagir avec le réactif (I) initiant i chaînes hydrophiles, selon le schéma décrit ci-dessus.

Les composés $R(OH)_i$ de formule (II) sont choisis parmi les alcools représentés par la formule générale ($II_1$)

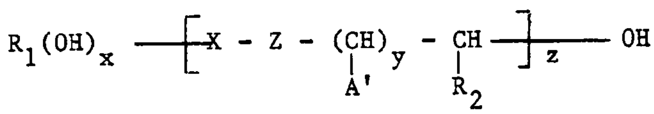

($II_1$)

où $R_1$, $R_2$, X, Z, x, y, z ont les significations ci-dessus indiquées et A′ désigne H ou OH.

Parmi ces alcools utilisables dans le procédé selon l'invention on peut citer à titre d'exemple :

les alcools aliphatiques d'origine naturelle ou les alcools de synthèse tels que ceux préparés par le procédé Ziegler, le procédé OXO ou le procédé Guerbet;

les alcools cycloaliphatiques comme les stérols tels que le cholestérol, le phytostérol ou le sitostérol, les alcools de lanoline ou les alcools résiniques;

les alcoylphénols, les alcools polyoxyéthylénés, les alcools cycloaliphatiques polyoxyéthylénés et les alcoylphénols polyoxyéthylénés, comportant de 1 à 20 motifs éthoxy;

les alcoylthio polyéthoxy éthanols, les alcoylcarbonyloxy (poly)éthoxy éthanols, les alcoylamido (poly)éthoxy éthanols, comportant de 1 à 20 motifs éthoxy;

les alcoyl-1 alcoyloxy-2-éthanols, les alcoxyméthyl-1 alcoyloxy-2 éthanols, les alcoyl-1 alcényloxy-2 éthanols, les alcoxyméthyl-1 alcényloxy-2 éthanols, les alcoyl-1 alcoylthio-2 éthanols, les alcoxyméthyl-1 alcoylthio-2 éthanols, les alcoyl-1 alcoylcarbonyloxy-2 éthanols, les alcoxyméthyl-1 alcoylcarbonyloxy-2 éthanols; les alcoyl-1 alcénylcarbonyloxy-2 éthanols, les alcoxyméthyl-1 alcénylcarbonylméthyl éthanols; les alcane diols-1,2, les alcoyléthers de glycérol, les alcoylthioéthers de glycérol et les alcoylesters de glycérol; les hydroxy-2 alcoyléthers de glycol, les hydroxy-2 alcoylthioéthers de glycol, les hydroxy-2 alcoyléthers de glycérol, les hydroxy-2 alcoylthioéthers de glycérol.

Les réactions de polyaddition de l'époxyde de formule (I) sur le composé hydroxylé (II) sont réalisées en présence de catalyseurs acides de Lewis tels que $BF_3$ ou $SnCl_4$ à une température comprise entre 20 et 80°C ou en présence de catalyseurs alcalins tels que le méthylate ou éthylate de sodium ou de potassium, ou le t-butylate de potassium, à une température comprise entre 125 et 155°C.

Les réactions sont généralement réalisées sans solvant mais des solvants comme le chlorure de méthylène, le chloroforme, l'heptane, le toluène, peuvent être utilisés avec les catalyseurs acides et des solvants comme le toluène, le xylène ou le cumène avec les catalyseurs alcalins.

L'époxyde (I) est généralement additionné progressivement à l'alcool $R(OH)_i$ auquel a été ajouté le catalyseur, en présence éventuellement d'un solvant, à la température de la réaction. Les durées d'addition sont généralement comprises entre 1 heure et 3 heures.

Une autre variante de la réaction, consiste à introduire rapidement tout l'époxyde à température ambiante et à chauffer très progressivement en évitant toute élévation brutale de température.

L'époxyde (I) est d'une façon générale miscible avec les alcools de formule (II) et donc les réactions d'addition se déroulent en phase homogène.

Le nombre n de moles d'époxyde (I) utilisé par mole de composé (II) est choisi en fonction de la structure de ce dernier et de l'hydrophilie souhaitée pour le produit (IV).

Le nombre n représente le nombre statistique moyen de moles d'époxyde (I) utilisé par mole d'alcool (II). Si ce dernier est polyfonctionnel, les n moles d'époxyde (I) sont réparties statistiquement entre les différents groupements hydroxyle.

Le mélange statistique de composés intermédiaires de formule générale (III) est ensuite hydrolysé, de préférence en solution hydro-alcoolique, en présence d'un catalyseur acide. Celui-ci peut être un acide minéral comme l'acide chlorhydrique, fluorhydrique, sulfurique ou phosphorique ou organique comme l'acide acétique ou lactique.

La réaction d'hydrolyse peut s'effectuer sans ajout de catalyseur acide lorsque la première étape de polyaddition a été réalisée en présence d'acide de Lewis tels que ceux mentionnés ci-dessus.

L'alcool utilisé comme cosolvant pour la réaction d'hydrolyse, peut être du méthanol, de l'éthanol ou de l'isopropanol. Après la réaction d'hydrolyse la masse réactionnelle est éventuellement neutralisée puis elle est chauffée sous pression réduite pour éliminer les produits volatils.

Le procédé de préparation sus-indiqué conduit à des produits non ioniques polyhydroxylés qui sont solubles ou dispersibles dans l'eau, selon la structure du composé II et le nombre de motifs hydrophiles.

Selon le cas, ces produits présentent des propriétés moussantes, émulsionnantes, dispersantes. Ils peuvent d'autre part être auto-émulsionnables c'est-à-dire se disperser facilement dans l'eau en donnant des dispersions laiteuses stables qui peuvent se présenter sous forme de microdispersions vésiculaires susceptibles éventuellement de véhiculer des substances actives.

Ces produits peuvent être utilisés comme tensio-actifs dans des compositions cosmétiques pour les soins de la peau et des cheveux, des compositions cosmétiques moussantes, comme les shampooings, les bains moussants, les compositions nettoyantes pour la peau; les laits ou les crèmes pour le visage ou pour le corps notamment les laits traitants pour le corps, les laits et crèmes adoucissants pour la peau, les laits et crèmes de soins pour le visage ou pour la peau, sous forme d'émulsion huile-dans-l'eau ou eau-dans-l'huile, de microémulsion, de microdispersion, de vésicules lipidiques contenant ou ne contenant pas de produits actifs; les compositions tinctoriales ou de coloration de la peau, les compositions solaires, les compositions adoucissantes ou démêlantes pour les cheveux. Ces compositions cosmétiques peuvent également se présenter sous forme de solution aqueuse, de lotions hydroalcooliques, de gels, de pains ou de produits à pulvériser.

Les produits tensio-actifs de l'invention peuvent être aisément associés aux différents constituants généralement présents dans les compositions cosmétiques ou pharmaceutiques.

L'invention a également pour objet les nouveaux composés tensio-actifs de fromule :

$$R\left[O\left[C_2H_3O\,(CH_2-O-CH_2-\underset{\displaystyle OH}{CH}-CH_2OH)\right]_{n/i}\right]_i H \qquad (X)$$

dans laquelle R désigne le radical :

$$\underset{(\;)_x}{\overset{R_1}{|}}\left[X-Z-(\underset{\displaystyle A}{CH})_y-\underset{\displaystyle R_2}{CH}\right]_z \qquad (VII)$$

où $R_1$, X, Z, A, $R_2$, x, y et z ont les significations ci-dessus indiqués,

i désigne 1, 2 ou 3

sous réserve que :

1) lorsque x=1, z=0 et $R_1$ représente un radical aliphatique, celui-ci comporte obligatoirement de 24 à 36

atomes de carbone et de préférence de 28 à 36 atomes de carbone.

2) Lorsque x = 0, z = 1, y = 1, A = H et X = CONH ou $CH_2$, alors le nombre total d'atomes de carbone dans $R_1$, Z et $R_2$ est de 23 à 35 et de préférence de 27 à 35.

3) Lorsque x = 0, z = 1, y = 1, A = liaison covalente et $R_2$ = H alors X = COO ou CONH.

L'invention a également pour objet les compositions contenant les nouveaux composés de formule (X).

Parmi ces compositions il faut citer les compositions cosmétiques, les compositions pharmaceutiques et les compositions utilisées pour les textiles.

Les compositions cosmétiques conviennent plus particulièrement au traitement des cheveux, de la peau et du cuir chevelu. Ces compositions peuvent comprendre des solvants et en particulier des solvants alcooliques, des tensio-actifs non ioniques autres que ceux de formule (X), des tensioactifs ioniques, des polymères naturels ou synthétiques, des huiles ou des cires minérales, animales ou végétales, des dérivés de polysiloxane, des propulseurs, des épaississants, des conservateurs, des filtres solaires, des colorants, des parfums, des coémulsionnants, des stérols, des agents hydratants, des produits actifs pour le traitement des affections de la peau ou du cuir chevelu, ainsi que les divers adjuvants habituellement utilisés dans les compositions cosmétiques et pharmaceutiques.

Ces compositions peuvent se présenter sous des formes diverses, notamment sous forme de solutions, de lotions hydroalcooliques, d'émulsions huile-dans-l'eau ou eau-dans-l'huile, de micro-émulsion, de microdispersion de vésicules lipidiques, renfermant facultativement des produits actifs éventuellement en présence d'huiles, de gels, de pains ou de produits à pulvériser.

L'invention a également pour objet l'utilisation des compositions renfermant un composé de formule (X) ou un produit résultant du procédé de préparation ci-dessus décrit, plus particulièrement dans les domaines cosmétiques, pharmaceutiques et textiles.

L'invention sera mieux comprise à l'aide des exemples non limitatifs ci-après.

## EXEMPLE 1

Préparation d'un mélange de composés de formule (IV) dans laquelle R désigne $C_{12}H_{25}$; i=1; n = 1,5

A 74,4 g (0,4 mole) de dodécanol-1 on ajoute 3,6 g de solution méthanolique de méthylate de sodium à 5,6 meq./g. On chauffe progressivement jusqu'à 150°C sous pression réduite pour éliminer le méthanol. On ajoute ensuite goutte à goutte à 140-145°C, sous léger courant d'azote, 112,8 g (0,6 mole) d'époxyde de formule (I). Durée de l'introduction 30 minutes. On maintient l'agitation et le chauffage pendant encore 2 heures.

La masse réactionnelle est chauffée ensuite sous pression réduite. On distille ainsi 12,7 g d'alcool non condensé. Le mélange de produits intermédiaires de formule (III) comporte donc statistiquement 1,8 motif hydrophile par chaîne grasse.

Ce mélange est repris dans 60 ml d'isopropanol et 10 ml d'eau, en présence de 4 ml d'acide chlorhydrique 11,6 N.

Après une nuit à la température ambiante, l'acide chlorhydrique est neutralisé avec NaOH et les solvants sont éliminés sous pression réduite.

Le produit obtenu se présente sous la forme d'une pâte molle de couleur ambre, parfaitement soluble dans l'eau et ayant de très bonnes propriétés moussantes.

Le point de trouble d'une solution à 0,5 % dans de l'eau à 25% de NaCl est de 86°C.

## EXEMPLE 2

Préparation d'un mélange de composés de formule (X) dans laquelle i=1, n=3, R désigne le radical $C_{12}H_{25}\ O-CH_2-\underset{\displaystyle R_2}{\underset{|}{CH}}-$

où $R_2$ désigne un mélange de radicaux $C_{14}H_{29}$ et $C_{16}H_{33}$ dans les proportions molaires 1/1.

A 44 g (0,1 mole) d'alcool ($II_1$), obtenu selon le brevet français 2 465 780, on ajoute 0,3 ml de complexe éthéré de trifluorure de bore puis, goutte à goutte, à 60°C, en 1 heure 15 minutes, 56,4 g (0,3 mole) d'époxyde de formule (I).

Le produit obtenu après 30 minutes de chauffage supplémentaires est fortement coloré. Il est repris avec 100 ml de méthanol et 20 ml d'eau, en présence de 4 ml d'acide chlorhydrique concentré.

Après une nuit à la température ambiante et évaporation des solvants, on obtient une pâte molle de couleur

brune dispersible dans l'eau.

Le point de trouble d'une solution à 5% dans un mélange de butyldiglycol à 25% dans l'eau est de 93°C.

EXEMPLE 3

Préparation d'un mélange de composés de formule (X) dans laquelle R désigne le reste hydrocarboné du cholestérol, i=1, n=3,

A 19,3 g (0,05 mole) de cholestérol on ajoute 35 g de toluène et 0,12 ml de complexe éthéré de $BF_3$ puis à la température de 50-60°C, en 1 heure 10 minutes 28,2 g (0,15 mole) d'époxyde (I).

On maintient la température et l'agitation pendant encore 15 minutes puis on élimine le toluène sous pression réduite. On reprend la masse réactionnelle avec 50 ml de méthanol, 7 ml d'eau et 2 ml d'acide chlorhydrique.

Après une nuit à la température ambiante, on élimine les solvants par chauffage sous pression réduite.

On obtient une pâte de couleur brun clair bien dispersible dans l'eau avec épaississement, donnant une dispersion translucide.

EXEMPLE 4

Préparation d'un mélange de composés de formule (X) dans laquelle R désigne le radical décyl-2-tétradécyle, i=1, n=3,

A 24,8 g (0,07 mole) de décyl-2-tétradécanol on ajoute 0,16 ml de complexe éthéré de $BF_3$, puis à la température de 50-60°C, en 1 heure, 39,5 g (0,21 mole) d'époxyde de formule (I).

La masse réactionnelle est reprise avec un mélange de 60 ml de méthanol, 10 ml d'eau et 4 ml d'acide chlorhydrique.

Après une nuit à la température ambiante on filtre la solution pour éliminer un très léger insoluble puis on distille les solvants sous pression réduite.

On obtient ainsi une pâte brune dispersible dans l'eau.

Le point de trouble d'une solution à 5% dans un mélange de butyldiglycol à 25% dans l'eau est de 99°C.

EXEMPLE 5

Preparation d'un mélange de composés de formule (X)dans laquelle R désigne le radical tétradécyl-2-octadécyle, i=1,n=5,

A 23,3 g de tétradécyl-2 octadécanol (0,05 mole) on ajoute 0,18 mole d'éthérate de $BF_3$ puis en 1 heure 30 minutes 47 g (0,25 mole) d'époxyde (I), à la température de 65-70°C.

Après 30 minutes d'agitation à 60°C, on reprend la masse réactionnelle avec 60 ml de méthanol, 10 ml d'eau et 4 ml d'acide chlorhydrique.

Après 16 heures à la température ambiante on neutralise l'acidité avec de la soude, on filtre l'insoluble et on distille les solvants sous pression réduite.

On obtient ainsi une pâte brune donnant à 10% une dispersion laiteuse dans l'eau.

EXEMPLE 6

Préparation d'un mélange de composés de formule (X) dans laquelle R désigne le radical hexadécyl-2 eicosyle, i=1, n=6,

A 26,1 g (0,05 mole) d'hexadécyl-2 eicosanol on ajoute 0,21 ml d'éthérate de $BF_3$ puis à 50-60°C en 1 heure 30 minutes, 56,4 g (0,3 mole) d'époxyde (I).

On maintient l'agitation et la température pendant 1 heure après l'addition. On reprend avec 50 ml de méthanol, 10 ml d'eau et 2 ml d'acide chlorhydrique. Après 20 heures à la température ambiante on neutralise, filtre l'insoluble et évapore les solvants sous pression réduite.

On obtient ainsi une pâte brune dispersible dans l'eau.

EXEMPLE 7

Préparation d'un mélange de composés de formule (X) dans laquelle R désigne un radical nonylphénoxy polyéthoxy éthyle à 9 motifs oxyéthyle, i=1, n=3,

A 30,8 g (0,05 mole) de nonylphénol polyoxyéthyléné à 9 moles d'oxyde d'éthylène on ajoute 0,6 g de méthylate de sodium à 5,56 meq./g. On élimine le méthanol en chauffant progressivement la masse réactionnelle jusqu'à 150°C sous courant d'azote. On introduit ensuite, goutte à goutte, en 1 heure 10 minutes, 28,2 g (0,15 mole) d'époxyde (1). Après l'addition on maintient le chauffage et l'agitation pendant 2 heures 30 minutes.

On reprend ensuite la masse réactionnelle avec 40 ml de méthanol et 10 ml d'eau en présence de 0,5 ml d'acide chlorhydrique concentré.

On laisse une nuit à température ambiante on neutralise l'acidité avec de la soude puis on distille les solvants sous pression réduite.

On obtient ainsi un produit qui se présente sous la forme d'une huile épaisse, de couleur brune, soluble dans l'eau.

EXEMPLE 8

Préparation d'un mélange de composés de formule (X) dans laquelle R désigne le radical bivalent $C_8H_{17}$-$CH_2$-$CH_2$-CH-$CH_2$-, dérivé du dodécanediol, i=2, n=1,5

A 101 g (0,5 mole) de dodécanediol-1,2 on ajoute 4,5 g de méthylate de sodium dans le méthanol (0,025 mole) puis on chauffe progressivement sous pression réduite jusqu'à 150°C pour éliminer complètement le méthanol.

On ajoute ensuite à pression ordinaire et à 135-140°C, 141 g d'époxyde (I) (0,75 mole).

Après 1 heure de chauffage après l'addition, on reprend la masse réactionnelle avec 150 ml d'isopropanol, 25 ml d'eau et 5 ml d'acide chlorhydrique.

Après une nuit à la température ambiante, on neutralise avec de la soude, puis on évapore les solvants sous pression réduite.

On obtient un produit qui se présente sous la forme d'une pâte de couleur beige clair. Le point de trouble d'une solution à 0,5 % dans une solution aqueuse de NaCl à 25% est supérieur à 100°C.

EXEMPLE 9

Préparation d'un mélange de composés de formule (IV) dans laquelle R désigne le radical stéaryle, i=1, n=3,5

A 16,2 g (0,06 mole) d'alcool stéarylique on ajoute 0,56 g de tétrachlorure d'étain, puis à la température de 55-60°C, goutte à goutte, 39,5 g (0,21 mole) d'époxyde de formule (I).

Durée de l'addition 1 heure. On maintient le chauffage et l'agitation pendant encore 30 minutes.

On obtient une huile épaisse de couleur brune que l'on reprend avec 40 ml de méthanol et 10 ml d'eau en présence de 0,5 ml d'acide chlorhydrique.

La solution obtenue est diluée avec 120 ml de méthanol et 70 ml d'eau et le mélange obtenu est traité avec de la résine échangeuse d'ion du type IRN 150, vendue par PROLABO, pendant 1 heure à la température du laboratoire.

Après filtration, les solvants sont évaporés sous pression réduite. On obtient ainsi une cire soluble dans l'eau au-dessus de 51°C. Le point de trouble mesuré à 0,5 % dans l'eau est supérieur à 100°C; dans une solution aqueuse à 10% de NaCl il est de 87°C.

EXEMPLE 10

Préparation d'un mélange de composés de formule (X) dans laquelle R désigne un radical divalent de formule $C_{17}H_{35}$-COO-$CH_2$-CH-$CH_2$-; i=2, n=3

A 34,4 g (0,1 mole) de monostéarate de glycérol vendu sous le nom de "TEGIN 90" par la société Goldschmidt, on ajoute 0,2 ml de complexe éthéré de BF3, puis goutte à goutte, à la température de 70-80°C, 56,4 g (0,3 mole) d'époxyde de formule (I). Durée de l'addition 1 heure. La masse réactionnelle se présente à chaud sous la forme d'une huile de couleur jaune figeant à froid.

On la solubilise dans 75 ml d'isopropanol et on ajoute 6 ml d'eau. On chauffe à 40-45°C pendant 5 heures. Par dilution à l'eau, la solution obtenue reste parfaitement limpide.

On évapore les solvants sous pression réduite. Le résidu se présente, à froid, sous la forme d'une pâte blanchâtre soluble dans l'eau avec une très légère opalescence.

## EXEMPLES D'APPLICATION

### EXEMPLE 11

On prépare le shampooing ayant la composition ci-après :

- Composés de l'exemple 8 12 g M.A.
- Gomme de xanthane vendue sous la dénominaton de "KELTROL T" par la société KELCO 0,5 g M.A.
- Chlorure de sodium 3 g
- Protéine quaternisée dénommée (dans le dictionnaire CTFA) "Cocotrimonium Collagen Hydrolysate", vendue sous la dénomination "LEXEIN QX 3000" par la Société INOLEX à la concentration de 30% M.A. 0,3 g M.A.
- Conservateur, parfum, colorant qs
- Eau qs 100 g

M.A. = Matière active

pH = 6,7 avec triéthanolamine.

Ce shampooing se présente sous la forme d'une solution épaissie opalescente produisant une mousse abondante; il facilite le démêlage des cheveux mouillés et le coiffage des cheveux séchés.

### EXEMPLE 12

On prépare le shampooing ayant la composition ci-après.

- Composés de l'exemple 1 10 g M.A.
- Cocoamphocarboxy glycinate vendu sous la dénomination "MIRANOL C2M conc. NP", à 38% M.A. par la Société MIRANOL 3 g M.A.
- Polyaminoamide résultant de la condensation de la diéthylène triamine et de l'acide adipique, réticulé par l'épichlorhydrine, à raison de 11 moles d'épichlorhydrine pour 100 groupements amine secondaire du polyaminoamide. 1 g M.A.
- Conservateur, parfum, colorant qs
- Eau qs 100 g
- pH = 8 avec acide chlorhydrique

Ce shampooing se présentant sous la forme d'une solution fluide, aux bonnes propriétés moussantes, apporte volume et nervosité aux cheveux séchés.

### EXEMPLE 13

On prépare le lait adoucissant pour le corps sous la forme d'une émulsion huile dans l'eau.

- Composés de l'exemple 3 10 g
- Huile de vaseline 40 g
- Eau qs 100 g

Le mélange de composés de l'exemple 3 est solubilisé dans l'huile de vaseline en chauffant jusqu'à 80-90°C. On refroidit vers 60°C et on ajoute l'eau, elle-même chauffée à 60°C, tout en agitant vigoureusement.

On obtient une émulsion huile-dans-l'eau qui s'affine (particules de l'ordre de grandeur de 1 µm) après traitement pendant 3 minutes aux ultrasons et qui est stable.

### EXEMPLE 14

On prépare le lait adoucissant ci-après sous la forme d'une émulsion eau dans-l'huile

- Composés de l'exemple 5 10 g
- Huile de vaseline 40 g

- Eau 50 g

En opérant comme dans l'exemple 13 on obtient une émulsion eau-dans-l'huile fine et stable.

EXEMPLE 15

On prépare une crème de soins pour le visage sous forme de dispersion vésiculaire, en procédant en deux étapes :

1ère étape

Dans un bécher en inox, on pèse les produits suivants :

- Composés de l'exemple 6 6 g
- Cholestérol 1,6 g
- Dicétylphosphate 0,4 g

On réalise le mélange de ces trois produits par fusion à la température de 110°C, sous atmosphère d'azote, puis on ramène la température du mélange fondu à 90°C.

| | |
|---|---|
| On ajoute alors de la glycérine | 3,0 g |
| dissoute dans l'eau déminéralisée | 20,0 g |

On homogénéise le mélange obtenu à la température de 90°C.

On ajoute alors

| | |
|---|---|
| parahydroxybenzoate de méthyle | 0,3 g |
| (stabilisateur) dissout dans l'eau | |
| déminéralisée | 22,5 g |

A la température de 70°C, on homogénéise le mélange à l'aide d'un ultradisperseur Virtis jusqu'à ce que la taille moyenne des vésicules obtenues soit de 0,3 micron.

2ème étape

| | |
|---|---|
| On ajoute l'huile de sésame | 25,0 g |

On soumet le tout à l'action de l'ultradisperseur Virtis jusqu'à ce que les globules d'huile formés aient un diamètre moyen inférieur au micron.

On ajoute enfin les substances suivantes :

- Parfum 0,4 g
- Mélange d'acides carboxyvinyliques commercialisé sous le nom de "CARBOPOL 940" 0,4 g
- Triéthanolamine 0,4 g
- Eau déminéralisée 20,0 g

EXEMPLE 16

On prépare un lait traitant pour le corps sous forme de dispersion vésiculaire.

1ère étape

Dans un bécher en inox, on pèse les produits suivants :

- Composés de l'exemple 5 0,95 g
- Dicétyl phosphate 0,05 g

On réalise le mélange de ces deux produits par fusion à la température de 110°C, sous atmosphère d'azote, puis on ramène la température du mélange fondu à 90°C.

| | |
|---|---|
| On ajoute alors de la glycérine | 1,0 g |
| dissoute dans l'eau déminéralisée | 3,0 g |

On homogénéise le mélange obtenu à la température de 90°C et on ajoute :

| | |
|---|---|
| Glycérine | 2,0 g |
| Parahydroxybenzoate de méthyle (stabilisateur) | 0,3 g |
| dans l'eau déminéralisée | 56,9 g |

On homogénéise le mélange à la température de 40°C, à l'aide d'un ultradisperseur Virtis jusqu'à ce que la taille moyenne des vésicules obtenues soit de 0,1 micron.

2ème étape

On ajoute alors :

| | |
|---|---|
| l'huile de silicone volatile | 15,0 g |

On soumet le tout à l'action d'un ultradisperseur Virtis jusqu'à ce que les globules d'huile formés aient un diamètre moyen inférieur au micron.

On ajoute enfin les substances suivantes :

- Parfum 0,4 g
- Mélange d'acides carboxyvinyliques, commecialisé sous le nom de "CARBOPOL 940" 0,2 g
- Triéthanolamine 0,2 g
- Eau déminéralisée 20,0 g

EXEMPLE 17

On prépare le shampooing ayant la composition ci-après :

- Composés de l'exemple 10 10 g M.A.
- Gomme de xanthane vendue sous la dénomination de "KELTROL T" par la société KELCO 0,5 g M.A.
- Chlorure de sodium 3 g
- Protéine quaternisé dénommée (dans le dictionnaire CTFA) "Cocotrimonium Collagen Hydrolysate", vendue sous la dénomination "LEXEIN QX 3000" par la Société INOLEX à la concentration de 30% M.A. 0,3 g M.A.
- Conservateur, parfum, colorant qs
- Eau qsp 100 g

M.A. = Matière active

pH = 6,7 avec triéthanolamine.

Ce shampooing se présente sous la forme d'une solution épaissie opalescente, il facilite le démêlage des cheveux mouillés et le coiffage des cheveux séchés.

## Revendications

### Revendications pour les Etats contractants suivants : AT, BE, CH, DE, DK, FR, GB, IT, LI, NL, SE

1. Procédé de préparation de produits tensio-actifs non ioniques à partir de l'isopropylidène-1,2 epoxypropyl-3 glycérol, en deux étapes, caractérisé par le fait que dans la première étape on fait réagir un composé de formule :

$$R(OH)_l \qquad (II)$$

comportant une ou plusieurs fonctions hydroxyle sur l'isopropylidène-1,2-époxypropyl-3 glycérol de formule (I), selon la réaction :

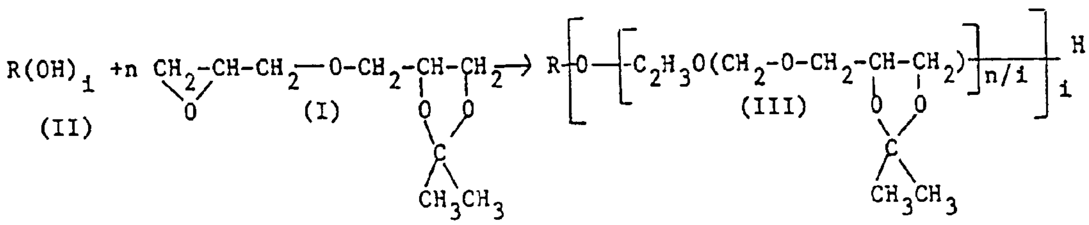

(II) (I) (III)

et que dans une deuxième étape on hydrolyse le composé intermédiaire de formule (III), à la température ambiante, en milieu hydroalcoolique en présence d'un catalyseur acide en obtenant les composés polyhydroxylés de formule (IV)

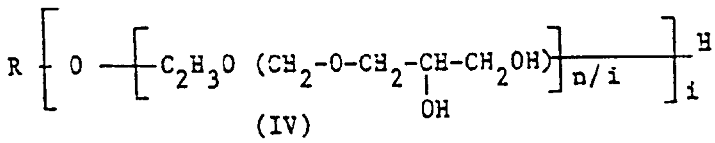

(IV)

Dans les formules ci-dessus,
le groupement

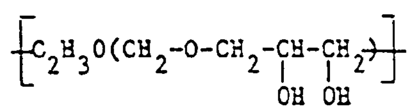

désigne l'un ou l'autre des deux groupement suivants, selon le sens d'ouverture de l'époxyde :

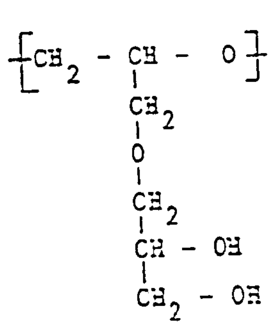

(V)

ou

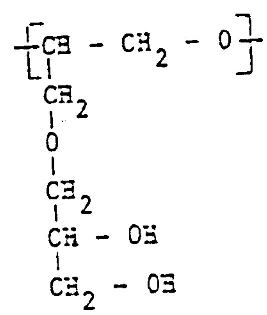

(VI)

i désigne 1,2 ou 3
n désigne un nombre entier ou décimal de 1,5 à 10
R désigne le radical

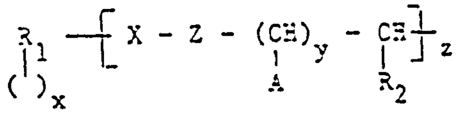 (VII)

dans laquelle

$R_1$ désigne un radical mono ou divalent, aliphatique, cycloaliphatique ou alcoylarylique en $C_8$-$C_{36}$,
$R_2$ désigne H, un radical alcoyle ou alcoxyméthyle, alcényle ou alcényloxyméthyle en $C_8$ à $C_{23}$,
X désigne 0, S, S(0), COO, CONH ou $CH_2$,
Z désigne $CH_2$ ou ($CH_2$-$CH_2$ O)m,
m est un nombre entier ou décimal de 1 à 20,
A désigne H ou une liaison covalente,
x, y et z désignent indépendamment l'un de l'autre 0 ou 1,
sous réserve que lorsque Z désigne $(CH_2CH_2O)_m$ alors y = 1 et A = $R_2$= H.

2. Procédé selon la revendication 1, caractérisé par le fait que la polyaddition est réalisée en présence d'un catalyseur acide à une température comprise entre 20 et 80°C.

3. Procédé selon la revendication 1, caractérisé par le fait que la polyaddition est réalisée en présence d'un catalyseur alcalin à une température comprise entre 125 et 155°C.

4. Procédé selon l'une quelconque des revendications 1 à 3, caractérisé par le fait que le composé de formule :

$$R(OH)_i \qquad (II)$$

est un alcool aliphatique d'origine naturelle ou synthétique, un alcool cycloaliphatique, un alcoylphénol, un alcool polyoxyéthyléné, un alcool cycloaliphatique polyoxyéthyléné ou un alcoylphénol polyoxyéthyléné, comportant de 1 à 20 motifs éthoxy, un alcoylthio polyéthoxyéthanol, un alcoyl carbonyloxy (poly)éthoxyéthanol, un acloylamido (poly)éthoxyéthanol, comportant de 1 à 20 motifs éthoxy, un alcoyl-1 alcoyloxy-2 éthanol, un alcoxyméthyl-1 alcoyloxy-2 éthanol, un alcoyl-1 alcényloxy-2 éthanol, un alcoxyméthyl- 1 alcényloxy-2 éthanol, un alcoyl-1 alcoylthio-2 éthanol, un alcoxyméthyl-1 alcoylthio-2 éthanol, un alcoyl-1 alcoylcarbonyloxy-2 éthanol, un alcoxyméthyl-1 alcoylcarbonyloxy-2 éthanol, un alcoyl-1 alcénylacarbonyloxy-2 éthanol, un alcoxyméthyl- 1 alcénylcarbonylméthyl-2 éthanol, un alcanediol-1,2, un alcoyléther de glycérol, un alcoylthio éther de glycérol, un alcoylester de glycérol, un hydroxy-2 alcoyléther de glycol, un hydroxy-2 alcoylthio éther de glycol, un hydroxy-2 alcoyléther de glycérol, un hydroxy-2 alcoylthioéther de glycérol.

5. Procédé selon l'une quelconque des revendications 1 à 4, caractérisé par le fait que l'époxyde (I) est additionné progressivement, en l'espace de 1 à 3 heures, à l'alcool (II) auquel a été ajouté le catalyseur, en présence ou en l'absence d'un solvant.

6. Procédé selon l'une quelconque des revendications 1 à 4, caractérisé par le fait que l'époxyde de formule (I) est ajouté, à la température ambiante, à l'alcool (II) contenant le catalyseur et qu'on chauffe très progressivement le mélange, en évitant une élévation brusque de la température.

7. Produit tensio-actif non ionique de formule :

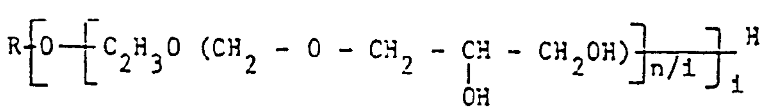 (X)

dans laquelle R désigne le radical :

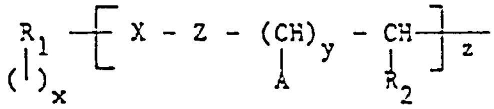 (VII)

où $R_1$, X, Z, $R_2$, x, y, n et z ont les significations indiquées dans la revendication 1, et
A désigne H ou une liaison covalente
i désigne 1, 2 ou 3
sous réserve que :

1) lorsque x = 1, z = 0 et $R_1$ représente un radical aliphatique, celui-ci comporte obligatoirement de 24 à 36 atomes de carbone, et de préférence de 28 à 36 atomes de carbone,
2) lorsque x = 0, z = 1, y = 1, A = H, et X = CONH ou $CH_2$ alors le nombre total d'atomes de carbone dans $R_1$, Z et $R_2$ est compris entre 23 et 35, et de préférence entre 27 et 35,
3) lorsque x = 0, z = 1, y = 1, A = liaison covalente et $R_2$ = H alors X = COO ou CONH.

**8.** Composition comprenant un ou plusieurs composés de formule (X) selon la revendication 7, dans un véhicule approprié.

**9.** Composition cosmétique ou pharmaceutique caractérisée par le fait qu'elle comprend un ou plusieurs composés de formule (X) dans un véhicule approprié.

**10.** Composition destinée à l'industrie textile comprenant un ou plusieurs composés de formule (X), selon la revendication 7 dans un véhicule approprié.

**11.** Composition selon la revendication 8 ou 9, caractérisée par le fait qu'elle renferme également un ou plusieurs produits choisis parmi les tensio-actifs non ioniques autres que ceux de formule (X), les tensio-actifs ioniques, les polymères naturels et synthétiques, les huiles et les cires minérales, animales ou végétales, les dérivés de polysiloxane, les solvants, les propulseurs, les épaississants, les conservateurs, les filtres solaires, les colorants, les parfums, les agents coémulsionnants, les stérols, les produits hydratants, les produits actifs pour le traitement des affections de la peau ou du cuir chevelu.

**12.** Composition selon l'une quelconque des revendications 8 à 11, caractérisée par le fait qu'elle se présente sous forme de solution aqueuse, de lotion hydroalcoolique, d'émulsion huile-dans-l'eau ou eau-dans-l'huile, de microémulsion, de microdispersion de vésicules lipidiques contenant ou ne contenant pas de produits actifs, de gel, de pain ou de produits à pulvériser.

**Revendications pour l'Etat contractant suivant : ES**

**1.** Procédé de préparation de produits tensio-actifs non ioniques à partir de l'isopropylidène-1,2 epoxypropyl-3 glycérol, en deux étapes, caractérisé par le fait que dans la première étape on fait réagir un composé de formule :

$$R(OH)_i \qquad (II)$$

comportant une ou plusieurs fonctions hydroxyle sur l'isopropylidène-1,2-époxypropyl-3 glycérol de formule (I), selon la réaction :

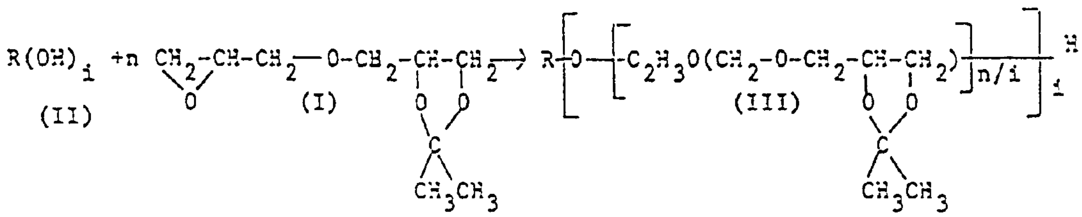

(II) (I) (III)

et que dans une deuxième étape on hydrolyse le composé intermédiaire de formule (III), à la température

ambiante, en milieu hydroalcoolique en présence d'un catalyseur acide en obtenant les composés polyhydroxylés de formule (IV)

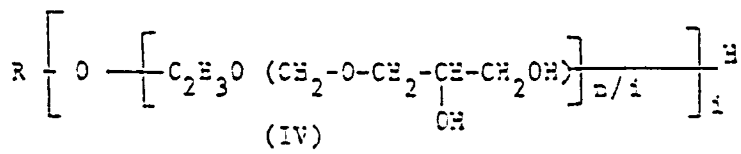

(IV)

Dans les formules ci-dessus,
le groupement

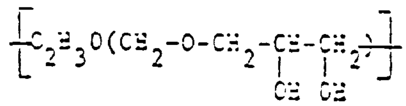

désigne l'un ou l'autre des deux groupement suivants, selon le sens d'ouverture de l'époxyde :

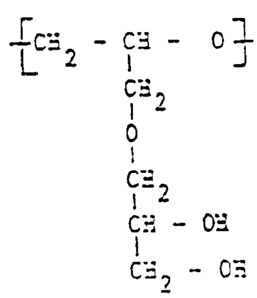

(V) ou

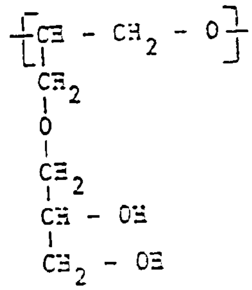

(VI)

i désigne 1,2 ou 3

n désigne un nombre entier ou décimal de 1,5 à 10

R désigne le radical

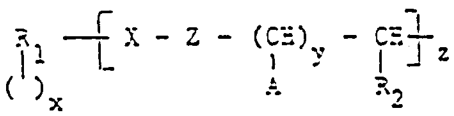

(VII)

dans laquelle

$R_1$ désigne un radical mono ou divalent, aliphatique. cycloaliphatique ou alcoylarylique en $C_8$-$C_{36}$,

$R_2$ désigne H, un radical alcoyle ou alcoxyméthyle, alcényle ou alcényloxyméthyle en $C_8$ à $C_{23}$,

X désigne 0, S. S(0), COO, CONH ou $CH_2$,

Z désigne $CH_2$ ou ($CH_2$-$CH_2$ O)m,

m est un nombre entier ou décimal de 1 à 20,

A désigne H ou une liaison covalente,

x, y et z désignent indépendamment l'un de l'autre 0 ou 1,

sous réserve que lorsque Z désigne $(CH_2CH_2O)_m$ alors y = 1 et A = $R_2$ = H.

2. Procédé selon la revendication 1, caractérisé par le fait que la polyaddition est réalisée en présence d'un catalyseur acide à une température comprise entre 20 et 80°C.

3. Procédé selon la revendication 1, caractérisé par le fait que la polyaddition est réalisée en présence d'un catalyseur alcalin à une température comprise entre 125 et 155°C.

4. Procédé selon l'une quelconque des revendications 1 à 3, caractérisé par le fait que le composé de formule :

$$R(OH)_l \qquad (II)$$

est un alcool aliphatique d'origine naturelle ou synthétique, un alcool cycloaliphatique, un alcoylphénol, un alcool polyoxyéthyléné, un alcool cycloaliphatique polyoxyéthyléné ou un alcoylphénol polyoxyéthyléné, comportant de 1 à 20 motifs éthoxy, un alcoylthio polyéthoxyéthanol, un alcoyl carbonyloxy (poly)éthoxyéthanol, un acloylamido (poly)éthoxyéthanol, comportant de 1 à 20 motifs éthoxy, un alcoyl-1 alcoyloxy-2 éthanol, un alcoxyméthyl-1 alcoyloxy-2 éthanol, un alcoyl-1 alcényloxy-2 éthanol, un alcoxyméthyl-1 alcényloxy-2 éthanol, un alcoyl-1 alcoylthio-2 éthanol, un alcoxyméthyl-1 alcoylthio-2 éthanol, un alcoyl-1 alcoylcarbonyloxy-2 éthanol, un alcoxyméthyl-1 alcoylcarbonyloxy-2 éthanol, un alcoyl-1 alcénylacarbonyloxy-2 éthanol, un alcoxyméthyl- 1 alcénylcarbonylméthyl-2 éthanol, un alcanediol-1,2, un alcoyléther de glycérol, un alcoylthio éther de glycérol, un alcoylester de glycérol, un hydroxy-2 alcoyléther de glycol, un hydroxy-2 alcoylthio éther de glycol, un hydroxy-2 alcoyléther de glycérol, un hydroxy-2 alcoylthioéther de glycérol.

5. Procédé selon l'une quelconque des revendications 1 à 4, caractérisé par le fait que l'époxyde (I) est additionné progressivement, en l'espace de 1 à 3 heures, à l'alcool (II) auquel a été ajouté le catalyseur, en présence ou en l'absence d'un solvant.

6. Procédé selon l'une quelconque des revendications 1 à 4, caractérisé par le fait que l'époxyde de formule (I) est ajouté, à la température ambiante, à l'alcool (II) contenant le catalyseur et qu'on chauffe très progressivement le mélange, en évitant une élévation brusque de la température.

7. Procédé de préparation de produits tensio-actifs non ioniques selon les revendications 1 à 6, caractérisé par le fait qu'on prépare des produits de formule :

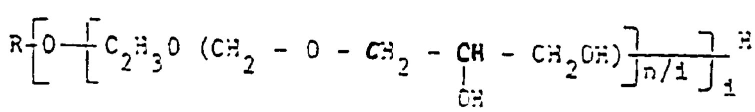

(X)

dans laquelle R désigne le radical :

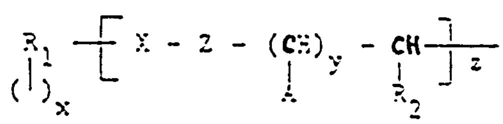

(VIII)

où $R_1$, X, Z, $R_2$, x, y, n et z ont les significations indiquées dans la revendication 1, et
A désigne H ou une liaison covalente
i désigne 1 2 ou 3
sous réserve que :
1) lorsque x = 1, z = 0 et $R_1$ représente un radical aliphatique, celui-ci comporte obligatoirement de 24 à 36 atomes de carbone, et de préférence de 28 à 36 atomes de carbone,
2) lorsque x = 0, z = 1, y = 1, A = H, et X = CONH ou $CH_2$ alors le nombre total d'atomes de carbone dans $R_1$, Z et $R_2$ est compris entre 23 et 35, et de préférence entre 27 et 35,
3) lorsque x = 0, z = 1, y = 1, A = liaison covalente et $R_2$ = H alors X = COO ou CONH.

8. Composition comprenant un ou plusieurs composés de formule (X) selon la revendication 7, dans un véhicule approprié.

**9.** Composition cosmétique caractérisée par le fait qu'elle comprend un ou plusieurs composés de formule (X) selon la revendication 7, dans un véhicule approprié.

**10.** Composition destinée à l'industrie textile comprenant un ou plusieurs composés de formule (X), selon la revendication 7 dans un véhicule approprié.

**11.** Composition selon la revendication 8 ou 9, caractérisée par le fait qu'elle renferme également un ou plusieurs produits choisis parmi les tensio-actifs non ioniques autres que ceux de formule (X), les tensioactifs ioniques, les polymères naturels et synthétiques, les huiles et les cires minérales, animales ou végétales, les dérivés de polysiloxane, les solvants, les propulseurs, les épaississants, les conservateurs, les filtres solaires, les colorants, les parfums, les agents coémulsionnants, les stérols, les produits hydratants, les produits actifs pour le traitement des affections de la peau ou du cuir chevelu.

**12.** Composition selon l'une quelconque des revendications 8 à 11, caractérisée par le fait qu'elle se présente sous forme de solution aqueuse, de lotion hydroalcoolique, d'émulsion huile-dans-l'eau ou eau-dans-l'huile, de microémulsion, de microdispersion de vésicules lipidiques contenant ou ne contenant pas de produits actifs, de gel, de pain ou de produits à pulvériser.

## Patentansprüche

### Patentansprüche für folgende Vertragsstaaten : AT, BE, CH, DE, DK, FR, GB, IT, LI, NL, SE

**1.** Verfahren zur Herstellung oberflächenaktiver nichtionischer Produkte aus 1,2-Isopropyliden-3-epoxipropylglycerin, in zwei Stufen,
dadurch **gekennzeichnet,** daß
man in der ersten Stufe eine Verbindung der Formel:

$$R(OH)_i \quad \text{(II)}$$

mit einer oder mehreren Hydroxylfunktionen mit 1,2-Isopropyliden-3-epoxipropylglycerin der Formel (I) gemäß der Reaktion:

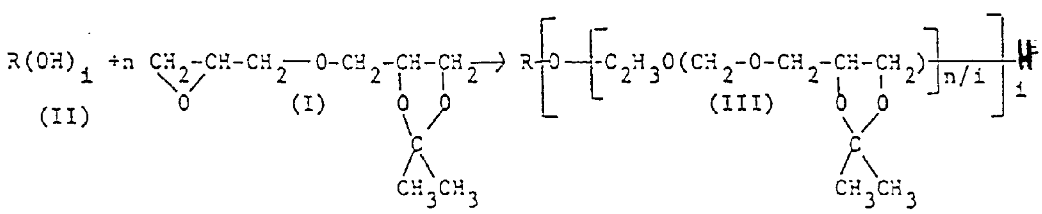
(II) (I) (III)

reagieren läßt,
und daß man in einer zweiten Stufe die Zwischenverbindung der Formel (III), bei Umgebungstemperatur, in hydroalkoholischem Medium in Gegenwart eines sauren Katalysators hydrolysiert, wobei man die polyhydroxylierten Verbindungen der Formel (IV) erhält:

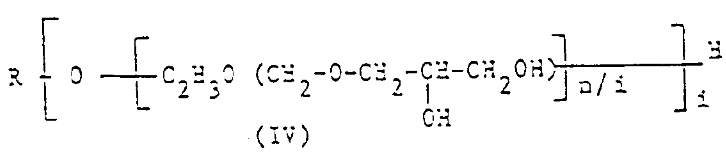
(IV)

worin in den obigen Formeln
die Gruppe:

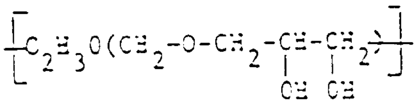

die eine oder die andere der beiden folgenden Gruppen bedeutet, gemäß der Richtung der Öffnung des Epoxids:

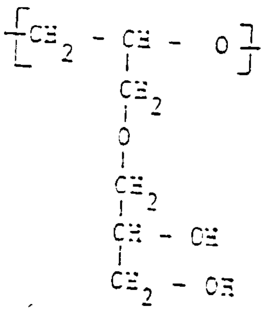

(V)

oder

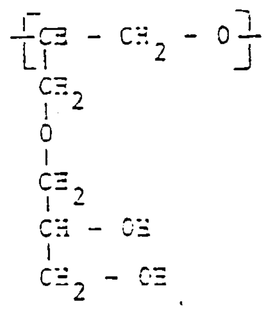

(VI)

i 1, 2 oder 3 ist,
n eine ganze Zahl oder Dezimalzahl von 1,5 bis 10 ist,
R den Rest

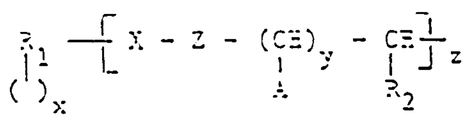

(VII)

darstellt, worin
$R_1$ einen ein- oder zweiwertigen aliphatischen, cycloaliphatischen oder Alkylarylrest, welche 8 bis 36 Kohlenstoffatome aufweisen,
$R_2$ H, einen Alkyl- oder Alkoxymethyl-, Alkenyl- oder Alkenyloxymethylrest mit 8 bis 23 Kohlenstoffatomen,
X 0, S, S(O), COO, CONH oder $CH_2$,
Z $CH_2$ oder $(CH_2\text{-}CH_2O)_m$ bedeuten,
worin m eine ganze Zahl oder Dezimalzahl von 1 bis 20 ist,
A H oder eine kovalente Bindung darstellt,
x, y und z, unabhängig voneinander, 0 oder 1 sind,
mit der Maßgabe, daß, wenn Z $(CH_2CH_2O)_m$ bedeutet, dann y = 1 und A = $R_2$ = H.

2. Verfahren gemäß Anspruch 1,
dadurch **gekennzeichnet,** daß
die Polyaddition in Gegenwart eines sauren Katalysators bei einer Temperatur von 20 bis 80°C durchgeführt wird.

3. Verfahren gemäß Anspruch 1,
dadurch **gekennzeichnet,** daß
die Polyaddition in Gegenwart eines alkalischen Katalysators bei einer Temperatur von 125 bis 155°C durchgeführt wird.

4. Verfahren gemäß jedem der Ansprüche 1 bis 3,
dadurch **gekennzeichnet,** daß
die Verbindung der Formel

$$R(OH)_i \qquad (II)$$

ein aliphatischer Alkohol natürlicher oder synthetischer Herkunft, ein cycloaliphatischer Alkohol, ein Alkylphenol, ein polyoxethylierter Alkohol, ein cycloaliphatischer polyoxethylieter Alkohol oder ein polyoxethyliertes Alkylphenol, welche 1 bis 20 Ethoxy-Einheiten enthalten, ein Alkylthiopolyethoxyethanol, ein Alkylcarbonyloxy(poly)ethoxyethanol, ein Alkylamido(poly)ethoxyethanol, welche 1 bis 20 Ethoxy-Einheiten enthalten, ein 1-Alkyl-2-alkyloxyethanol, ein 1-Alkoxymethyl-2-alkyloxyethanol, ein 1-Alkyl-2-alkenyloxyethanol, ein 1-Alkoxymethyl-2-alkenyloxyethanol, ein 1-Alkyl-2-alkylthioethanol, ein 1-Alkoxymethyl-2-alkylthioethanol, ein 1-Alkyl-2-alkylcarbonyloxyethanol, ein 1-Alkoxymethyl-2-alkylcarbonyloxyethanol, ein 1-Alkyl-2-alkenylcarbonyloxyethanol, ein 1-Alkoxymethyl-2-alkenylcarbonylmethylethanol, ein 1,2-Alkandiol, ein Alkylether von Glycerin, ein Alkylthioether von Glycerin, ein Alkylester von Glycerin, ein 2-Hydroxyalkylether von Glycol, ein 2-Hydroxyalkylthioether von Glycol, ein 2-Hydroxyalkylether von Glycerin, ein 2-Hydroxyalkylthioether von Glycerin ist.

5. Verfahren gemäß jedem der Ansprüche 1 bis 4,
dadurch **gekennzeichnet**, daß
das Epoxid (I) fortschreitend, während 1 bis 3 Stunden, an den Alkohol (II), dem der Katalysator zugefügt worden ist, in Gegenwart oder Abwesenheit eines Lösungsmittels addiert wird.

6. Verfahren gemäß jedem der Ansprüche 1 bis 4,
dadurch **gekennzeichnet**, daß
das Epoxid der Formel (I), bei Umgebungstemperatur, dem Alkohol (II) zugefügt wird, der den Katalysator enthält, und daß maß die Mischung sehr langsam erwärmt, wobei man eine sprunghafte Steigerung der Temperatur vermeidet.

7. Oberflächenaktives nicht-ionisches Produkt der Formel:

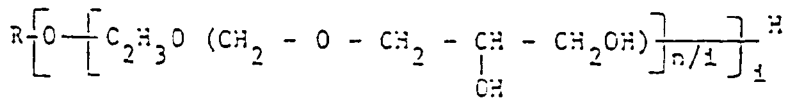 (X)

worin R den Rest:

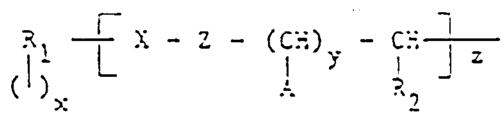 (VII),

darstellt,
worin $R_1$, X, Z, $R_2$, x, y, n und z die in Anspruch 1 angegebenen Bedeutungen haben, und
A H oder eine kovalente Bindung bedeutet,
i 1, 2 oder 3 ist,
mit der Maßgabe, daß:
1) wenn x = 1, z = 0 und $R_1$ einen aliphatischen Rest darstellt, dieser zwingend 24 bis 36 und vorzugsweise 28 bis 36 Kohlenstoffatome umfaßt,
2) wenn x = 0, z = 1, y = 1, A = H und X = CONH oder $CH_2$, dann die Gesamtzahl an Kohlenstoffatomen in $R_1$, Z und $R_2$ 23 bis 35 und vorzugsweise 27 bis 35 beträgt,
3) wenn x = 0, z = 1, y = 1, A = eine kovalente Bindung und $R_2$ = H, dann X = COO oder CONH.

8. Zusammensetzung, enthaltend eine oder mehrere Verbindungen der Formel (X) gemäß Anspruch 7 in einem geeigneten Träger.

9. Kosmetische oder pharmazeutische Zusammensetzung,
dadurch **gekennzeichnet**, daß
sie eine oder mehrere Verbindungen der Formel (X) gemäß Anspruch 7 in einem geeigneten Träger enthält.

10. Zusammensetzung für die Textilindustrie, enthaltend eine oder mehrere Verbindungen der Formel (X) gemäß Anspruch 7 in einem geeigneten Träger.

11. Zusammensetzung gemäß Anspruch 8 oder 9,
dadurch **gekennzeichnet,** daß
sie auch ein oder mehrere Produkte enthält, ausgewählt aus anderen oberflächenaktiven nicht-ionischen Mitteln als denjenigen der Formel (X), aus ionischen oberflächenaktiven Mitteln, natürlichen oder synthetischen Polymeren, mineralischen, tierischen oder pflanzlichen Ölen und Wachsen, Polysiloxanderivaten, Lösungsmitteln, Treibmitteln, Verdickungsmitteln, Konservierungsstoffen, Sonnenfilterstoffen, Farbstoffen, Parfüm-Produkten, Co-Emulgatoren, Sterolen, hydratisierenden Produkten, Wirkstoffprodukten zur Behandlung von Krankheiten der Haut oder behaarten Haut.

12. Zusammensetzung gemäß jedem der Ansprüche 8 bis 11,
dadurch **gekennzeichnet,** daß
sie in Form einer wässrigen Lösung, hydroalkoholischen Lotion, Öl-in-Wasser- oder Wasser-in-Öl-Emulsion, Mikroemulsion, Mikrodispersion lipidischer Bläschen, welche Wirkstoffprodukte enthalten oder nicht enthalten, eines Gels, einer Seife oder von Produkten zur Pulverisierung vorliegt.

**Patentansprüche für folgenden Vertragsstaat : ES**

1. Verfahren zur Herstellung oberflächenaktiver nichtionischer Produkte aus 1,2-Isopropyliden-3-epoxipropylglycerin, in zwei Stufen,
dadurch **gekennzeichnet,** daß
man in der ersten Stufe eine Verbindung der Formel:

$$R(OH)_l \quad \text{(II)}$$

mit einer oder mehreren Hydroxylfunktionen mit 1,2-Isopropyliden-3-epoxipropylglycerin der Formel (I) gemäß der Reaktion:

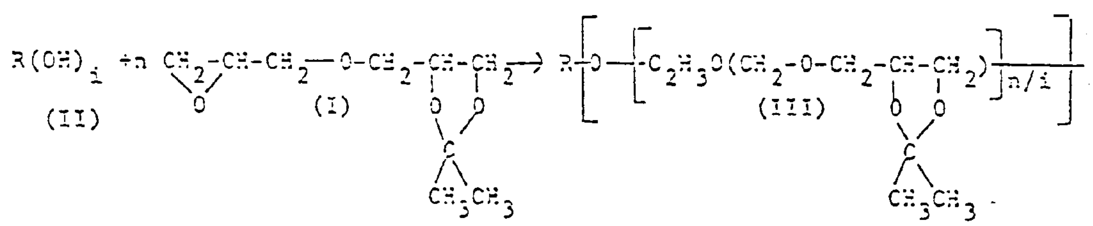
(II) (I) (III)

reagieren läßt,
und daß man in einer zweiten Stufe die Zwischenverbindung der Formel (III), bei Umgebungstemperatur, in hydroalkoholischem Medium in Gegenwart eines sauren Katalysators hydrolysiert, wobei man die polyhydroxylierten Verbindungen der Formel (IV) erhält:

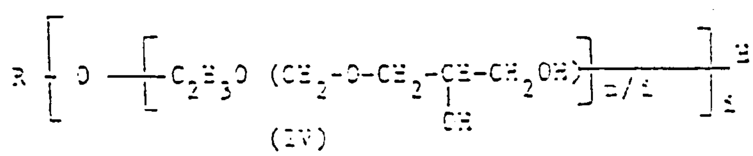
(IV)

worin in den obigen Formeln
die Gruppe:

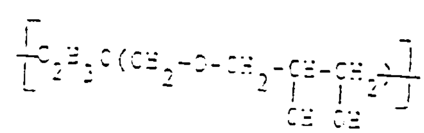

die eine oder die andere der beiden folgenden Gruppen bedeutet, gemäß der Richtung der Öffnung des

Epoxids:

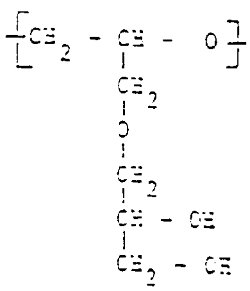 (V) oder 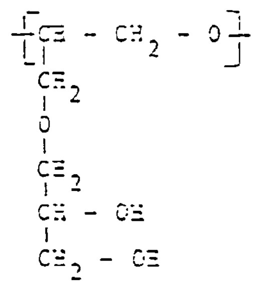 (VI)

i 1, 2 oder 3 ist,
n eine ganze Zahl oder Dezimalzahl von 1,5 bis 10 ist,
R den Rest

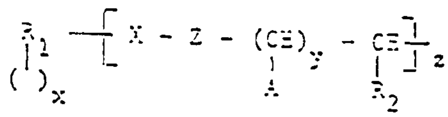 (VII)

darstellt, worin
$R_1$ einen ein- oder zweiwertigen aliphatischen, cycloaliphatischen oder Alkylarylrest, welche 8 bis 36 Kohlenstoffatome aufweisen,
$R_2$ H, einen Alkyl- oder Alkoxymethyl-, Alkenyl- oder Alkenyloxymethylrest mit 8 bis 23 Kohlenstoffatomen,
X 0, S, S(O), COO, CONH oder $CH_2$,
Z $CH_2$ oder $(CH_2\text{-}CH_2O)_m$ bedeuten,
worin m eine ganze Zahl oder Dezimalzahl von 1 bis 20 ist, A H oder eine kovalente Bindung darstellt,
x, y und z, unabhängig voneinander, 0 oder 1 sind,
mit der Maßgabe, daß, wenn Z $(CH_2CH_2O)_m$ bedeutet, dann y = 1 und A = $R_2$ = H.

2. Verfahren gemäß Anspruch 1,
dadurch **gekennzeichnet,** daß
die Polyaddition in Gegenwart eines sauren Katalysators bei einer Temperatur von 20 bis 80°C durchgeführt wird.

3. Verfahren gemäß Anspruch 1,
dadurch **gekennzeichnet**, daß
die Polyaddition in Gegenwart eines alkalischen Katalysators bei einer Temperatur von 125 bis 155°C durchgeführt wird.

4. Verfahren gemäß jedem der Ansprüche 1 bis 3,
dadurch **gekennzeichnet**, daß
die Verbindung der Formel

$$R(OH)_i \qquad (II)$$

ein aliphatischer Alkohol natürlicher oder synthetischer Herkunft, ein cycloaliphatischer Alkohol, ein Alkylphenol, ein polyoxethylierter Alkohol, ein cycloaliphatischer polyoxethylieter Alkohol oder ein polyoxethyliertes Alkylphenol, welche 1 bis 20 Ethoxy-Einheiten enthalten, ein Alkylthiopolyethoxyethanol, ein Alkylcarbonyloxy(poly)ethoxyethanol, ein Alkylamido(poly)ethoxyethanol, welche 1 bis 20 Ethoxy-Einheiten enthalten, ein 1-Alkyl-2-alkyloxyethanol, ein 1-Alkoxymethyl-2-alkyloxyethanol, ein 1-Alkyl-2-alkenyloxyethanol, ein 1-Alkoxymethyl-2-alkenyloxyethanol, ein 1-Alkyl-2-alkylthioethanol, ein 1-Alkoxymethyl-2-alkylthioethanol, ein 1-Alkyl-2-alkylcarbonyloxyethanol, ein 1-Alkoxymethyl-2-alkylcarbonyloxyethanol, ein 1-Alkyl-2-alkenylcarbonyloxyethanol, ein 1-Alkoxymethyl-2-alkenylcarbonylmethylethanol, ein 1,2-Alkandiol, ein Alkylether von Glycerin, ein Alkylthioether von Glycerin, ein Alkylester von Glycerin, ein 2-Hydroxyalkylether von Glycol, ein 2-Hydroxyalkylthioether von Glycol, ein 2-Hydroxyalkylether von Glycerin, ein 2-Hydroxyalkylthioether von Glycerin ist.

5. Verfahren gemäß jedem der Ansprüche 1 bis 4,
dadurch **gekennzeichnet**, daß
das Epoxid (I) fortschreitend, während 1 bis 3 Stunden, an den Alkohol (II), dem der Katalysator zugefügt worden ist, in Gegenwart oder Abwesenheit eines Lösungsmittels addiert wird.

6. Verfahren gemäß jedem der Ansprüche 1 bis 4,
dadurch **gekennzeichnet**, daß
das Epoxid der Formel (I), bei Umgebungstemperatur, dem Alkohol (II) zugefügt wird, der den Katalysator enthält, und daß maß die Mischung sehr langsam erwärmt, wobei man eine sprunghafte Steigerung der Temperatur vermeidet.

7. Verfahren zur Herstellung oberflächenaktiver nichtionischer Produkte gemäß den Ansprüchen 1 bis 6,
dadurch **gekennzeichnet**, daß
man Produkte der Formel herstellt:

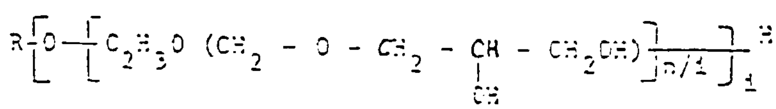

(X)

worin R den Rest:

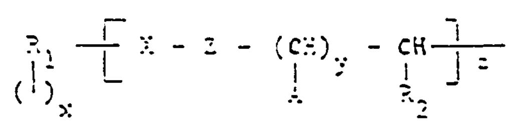

(VII)

darstellt,
worin $R_1$, X, Z, $R_2$, x, y, n und z die in Anspruch 1 angegebenen Bedeutungen haben, und
A H oder eine kovalente Bindung bedeutet,
i 1, 2 oder 3 ist,
mit der Maßgabe, daß:
1) wenn $x = 1$, $z = 0$ und $R_1$ einen aliphatischen Rest darstellt, dieser zwingend 24 bis 36 und vorzugsweise 28 bis 36 Kohlenstoffatome umfaßt,
2) wenn $x = 0$, $z = 1$, $y = 1$, A = H und X = CONH oder $CH_2$, dann die Gesamtzahl an Kohlenstoffatomen in $R_1$, Z und $R_2$ 23 bis 35 und vorzugsweise 27 bis 35 beträgt,
3) wenn $x = 0$, $z = 1$, $y = 1$, A = eine kovalente Bindung und $R_2$ = H, dann X = COO oder CONH.

8. Zusammensetzung, enthaltend eine oder mehrere Verbindungen der Formel (X) gemäß Anspruch 7 in einem geeigneten Träger.

9. Kosmetische Zusammensetzung,
dadurch **gekennzeichnet**, daß
sie eine oder mehrere Verbindungen der Formel (X) gemäß Anspruch 7 in einem geeigneten Träger enthält.

10. Zusammensetzung für die Textilindustrie, enthaltend eine oder mehrere Verbindungen der Formel (X) gemäß Anspruch 7 in einem geeigneten Träger.

11. Zusammensetzung gemäß Anspruch 8 oder 9,
dadurch **gekennzeichnet**, daß
sie auch ein oder mehrere Produkte enthält, ausgewählt aus anderen oberflächenaktiven nicht-ionischen Mitteln als denjenigen der Formel (X), aus ionischen oberflächenaktiven Mitteln, natürlichen oder synthetischen Polymeren, mineralischen, tierischen oder pflanzlichen Ölen und Wachsen, Polysiloxanderivaten, Lösungsmitteln, Treibmitteln, Verdickungsmitteln, Konservierungsstoffen, Sonnenfilterstoffen, Farbstoffen, Parfüm-Produkten, Co-Emulgatoren, Sterolen, hydratisierenden Produkten, Wirkstoffprodukten zur Behandlung von Krankheiten der Haut oder behaarten Haut.

**12.** Zusammensetzung gemäß jedem der Ansprüche 8 bis 11,
dadurch **gekennzeichnet**, daß
sie in Form einer wässrigen Lösung, hydroalkoholischen Lotion, Öl-in-Wasser- oder Wasser-in-Öl-Emulsion, Mikroemulsion, Mikrodispersion lipidischer Bläschen, welche Wirkstoffprodukte enthalten oder nicht enthalten, eines Gels, einer Seife oder von Produkten zur Pulverisierung vorliegt.

## Claims

### Claims for the following Contracting States : AT, BE, CH, DE, DK, FR, GB, IT, LI, NL, SE

**1.** Process for preparing nonionic surfactant products from 1,2-isopropylidene-3-epoxypropylglycerol, in two steps, characterized in that, in the first step, a compound of formula:

$$R(OH)_l \qquad (II)$$

containing one or more hydroxyl groups, is reacted with 1,2-isopropylidene-3-epoxypropylglycerol of formula (I), according to the reaction:

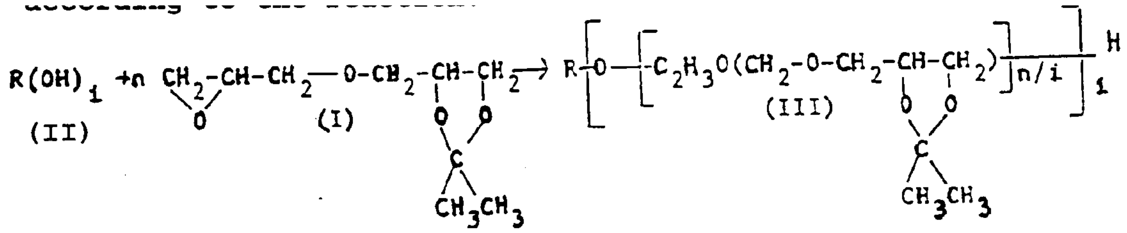

and in that, in a second step, the intermediate compound of formula (III) is hydrolysed at room temperature in an aqueous-alcoholic medium in the presence of an acid catalyst, the polyhydroxylated compounds of formula (IV)

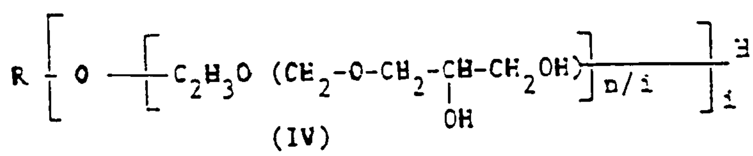

being obtained.
In the above formulae,

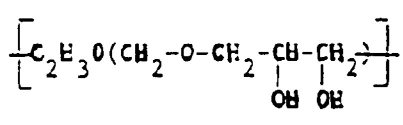

the group
denotes one or other of the following two groups, depend- ing on the direction of opening of the epoxide:

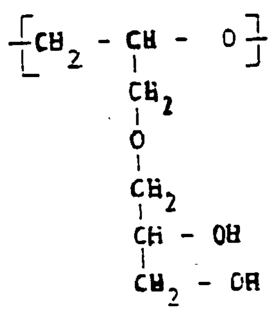 (V) or 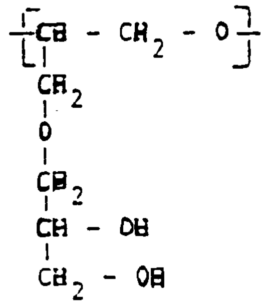 (VI)

i denotes 1, 2 or 3
n denotes an integer or decimal number from 1.5 to 10,
R denotes a radical

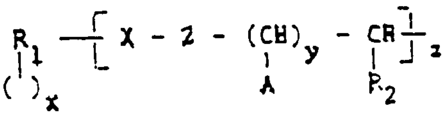 (VII)

in which

$R_1$ denotes a $C_8$-$C_{36}$ mono- or divalent aliphatic, cycloaliphatic or alkylaryl radical,
$R_2$ denotes H or a $C_8$ to $C_{23}$ alkyl, alkoxymethyl, alkenyl or alkenyloxymethyl radical,
X denotes O, S, S(O), COO, CONH or $CH_2$,
Z denotes $CH_2$ or $(CH_2$-$CH_2O)_m$,
m is an integer or decimal number from 1 to 20,
A denotes H or a covalent bond, and
x, y and z denote, independently of one another, 0 or 1,
with the proviso that when Z denotes $(CH_2CH_2O)_m$, then y = 1 and A = $R_2$ = H.

2. Process according to Claim 1, characterized in that the polyaddition is carried out in the presence of an acid catalyst at a temperature of between 20 and 80°C.

3. Process according to Claim 1, characterized in that the polyaddition is carried out in the presence of an alkaline catalyst at a temperature of between 125 and 155°C.

4. Process according to any one of Claims 1 to 3, characterized in that the compound of formula:

$$R(OH)_i \qquad (II)$$

is an aliphatic alcohol of natural or synthetic origin, a cycloaliphatic alcohol, an alkylphenol, a polyoxyethylenated alcohol, a polyoxyethylenated cycloaliphatic alcohol or a polyoxyethylenated alkylphenol, containing from 1 to 20 ethoxy units, an alkylthio(polyethoxy)ethanol, an alkylcarbonyloxy[(poly)ethoxy]ethanol, an alkylamido[(poly)ethoxy]ethanol, containing from 1 to 20 ethoxy units, a 1-alkyl-2-(alkyloxy)ethanol, a 1-alkoxy- methyl-2-(alkyloxy)ethanol, a 1-alkyl-2-(alkenyloxy)- ethanol, a 1-alkoxymethyl-2-(alkenyloxy)ethanol, a 1-alkyl-2-(alkylthio)ethanol, a 1-alkoxymethyl-2-(alkylthio)ethanol, a 1-alkyl-2-(alkylcarbonyloxy)ethanol, a 1-alkoxymethyl-2-(alkylcarbonyloxy)ethanol, a 1-alkyl-2- (alkenylcarbonyloxy)ethanol, a 1-alkoxymethyl-2-(alkenylcarbonylmethyl)ethanol, a 1,2-alkanediol, a glycerol alkyl ether, a glycerol alkyl thioether, a glycerol alkyl ester, a glycol 2-hydroxyalkyl ether, a glycol 2-hydroxyalkyl thioether, a glycerol 2-hydroxyalkyl ether or a glycerol 2-hydroxyalkyl thioether.

5. Process according to any one of Claims 1 to 4, characterized in that the epoxide (I) is added gradually, in the space of 1 to 3 hours, to the alcohol (II) to which the catalyst has been added, in the presence or absence of a solvent.

6. Process according to any one of Claims 1 to 4, characterized in that the epoxide of formula (I) is added at room temperature to the alcohol (II) containing the catalyst and in that the mixture is heated very gradually, avoiding a sudden temperature rise.

7. Nonionic surfactant product of formula:

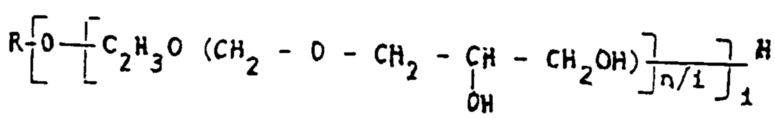 (X)

in which R denotes a radical:

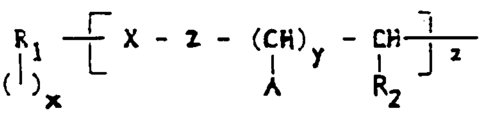 (VII)

where $R_1$, X, Z, $R_2$, x, y, n and z have the meanings stated in Claim 1, and
A denotes H or a covalent bond,
i denotes 1, 2 or 3,
with the proviso that:
1) when x = 1, z = 0 and $R_1$ represents an aliphatic radical, the latter necessarily contains from 24 to 36 carbon atoms, and preferably from 28 to 36 carbon atoms,
2) when x = 0, z = 1, y = 1, A = H and X = CONH or $CH_2$, the total number of carbon atoms in $R_1$, Z and $R_2$ is then between 23 and 35, and preferably between 27 and 35,
3) when x = 0, z = 1, y = 1, A = covalent bond and $R_2$ = H, then X = COO or CONH.

8. Composition comprising one or more compounds of formula (X) according to Claim 7, in a suitable vehicle.

9. Cosmetic or pharmaceutical composition, characterized in that it comprises one or more compounds of formula (X) in a suitable vehicle.

10. Composition intended for the textile industry, comprising one or more compounds of formula (X), according to Claim 7, in a suitable vehicle.

11. Composition according to Claim 8 or 9, characterized in that it also contains one or more products selected from nonionic surfactants other than those of formula (X), ionic surfactants, natural and synthetic polymers, mineral, animal or vegetable oils or waxes, polysiloxane derivatives, solvents, propellants, thickeners, preservatives, sunscreens, colourings, fragrances, coemulsifying agents, sterols, moisturizing products and active products for the treatment of skin or scalp conditions.

12. Composition according to any one of Claims 8 to 11, characterized in that it takes the form of an aqueous solution, aqueous-alcoholic lotion, oil-in-water or water-in-oil emulsion, microemulsion, microdispersion of lipid vesicles containing or not containing active products, gel or bar or of products for spraying.

**Claims for the following Contracting State : ES**

1. Process for preparing nonionic surfactant products from 1,2-isopropylidene-3-epoxypropylglycerol, in two steps, characterized in that, in the first step, a compound of formula:

$$R(OH)_i \qquad (II)$$

containing one or more hydroxyl groups, is reacted with 1,2-isopropylidene-3-epoxypropylglycerol of formula (I), according to the reaction:

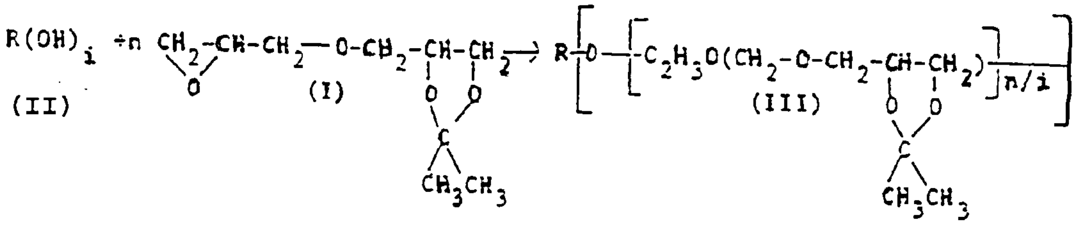

(II) (I) (III)

and in that, in a second step, the intermediate compound of formula (III) is hydrolysed at room temperature in an aqueous-alcoholic medium in the presence of an acid catalyst, the polyhydroxylated compounds of formula (IV)

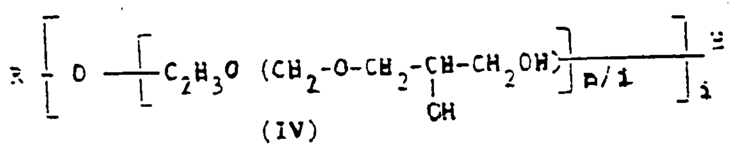

(IV)

being obtained.
In the above formulae,
the group

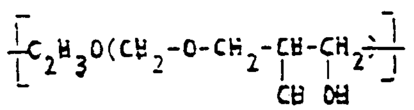

denotes one or other of the following two groups, depending on the direction of opening of the epoxide:

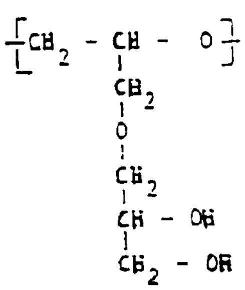

(V)

or

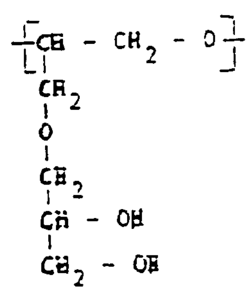

(VI)

i denotes 1, 2 or 3
n denotes an integer or decimal number from 1.5 to 10,
R denotes a radical

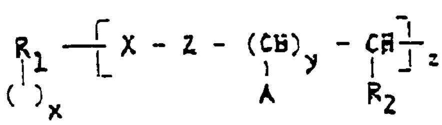

(VII)

in which
$R_1$ denotes a $C_8$-$C_{36}$ mono- or divalent aliphatic, cycloaliphatic or alkylaryl radical,
$R_2$ denotes H or a $C_8$ to $C_{23}$ alkyl, alkoxymethyl, alkenyl or alkenyloxymethyl radical,
X denotes O, S, S(O), COO, CONH or $CH_2$,

Z denotes $CH_2$ or $(CH_2\text{-}CH_2O)_m$,
m is an integer or decimal number from 1 to 20,
A denotes H or a covalent bond, and
x, y and z denote, independently of one another, 0 or 1,
with the proviso that when Z denotes $(CH_2CH_2O)_m$, then y = 1 and A = $R_2$ = H.

2. Process according to Claim 1, characterized in that the polyaddition is carried out in the presence of an acid catalyst at a temperature of between 20 and 80°C.

3. Process according to Claim 1, characterized in that the polyaddition is carried out in the presence of an alkaline catalyst at a temperature of between 125 and 155°C.

4. Process according to any one of Claims 1 to 3, characterized in that the compound of formula:

$$R(OH)_i \qquad (II)$$

is an aliphatic alcohol of natural or synthetic origin, a cycloaliphatic alcohol, an alkylphenol, a polyoxyethylenated alcohol, a polyoxyethylenated cycloaliphatic alcohol or a polyoxyethylenated alkylphenol, containing from 1 to 20 ethoxy units, an alkylthio(polyethoxy)ethanol, an alkylcarbonyloxy[(poly)ethoxy]ethanol, an alkylamido[(poly)ethoxy]ethanol, containing from 1 to 20 ethoxy units, a 1-alkyl-2-(alkyloxy)ethanol, a 1-alkoxy- methyl-2-(alkyloxy)ethanol, a 1-alkyl-2-(alkenyloxy)ethanol, a 1-alkoxymethyl-2-(alkenyloxy)ethanol, a 1-alkyl-2-(alkylthio)ethanol, a 1-alkoxymethyl-2-(alkylthio)ethanol, a 1-alkyl-2-(alkylcarbonyloxy)ethanol, a 1-alkoxymethyl-2-(alkylcarbonyloxy)ethanol, a 1-alkyl-2-(alkenylcarbonyloxy)ethanol, a 1-alkoxymethyl-2-(alkenyl carbonylmethyl)ethanol, a 1,2-alkanediol, a glycerol alkyl ether, a glycerol alkyl thioether, a glycerol alkyl ester, a glycol 2-hydroxyalkyl ether, a glycol 2-hydroxyalkyl thioether, a glycerol 2-hydroxyalkyl ether or a glycerol 2-hydroxyalkyl thioether.

5. Process according to any one of Claims 1 to 4, characterized in that the epoxide (I) is added gradually, in the space of 1 to 3 hours, to the alcohol (II) to which the catalyst has been added, in the presence or absence of a solvent.

6. Process according to any one of Claims 1 to 4, characterized in that the epoxide of formula (I) is added at room temperature to the alcohol (II) containing the catalyst and in that the mixture is heated very gradually, avoiding a sudden temperature rise.

7. Process for preparing nonionic surfactant products according to Claims 1 to 6, characterized in that products of formula:

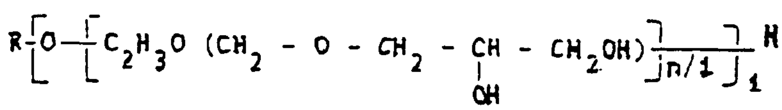

(X)

are prepared, in which R denotes a radical:

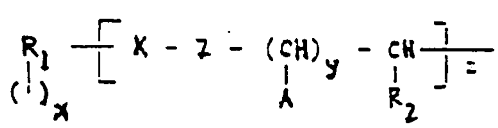

(VII)

where $R_1$, X, Z, $R_2$, x, y, n and z have the meanings stated in Claim 1, and
A denotes H or a covalent bond,
i denotes 1, 2 or 3,
with the proviso that:
1) when x = 1, z = 0 and $R_1$ represents an aliphatic radical, the latter necessarily contains from 24 to 36 carbon atoms, and preferably from 28 to 36 carbon atoms,
2) when x = 0, z = 1, y = 1, A = H and X = CONH or $CH_2$, the total number of carbon atoms in $R_1$, Z and $R_2$ is then between 23 and 35, and preferably between 27 and 35,

3) when x = 0, z = 1, y = 1, A = covalent bond and $R_2$ = H, then X = COO or CONH.

**8.** Composition comprising one or more compounds of formula (X) according to Claim 7, in a suitable vehicle.

**9.** Cosmetic composition characterized in that it comprises one or more compounds of formula (X) according to Claim 7, in a suitable vehicle.

**10.** Composition intended for the textile industry, comprising one or more compounds of formula (X), according to Claim 7, in a suitable vehicle.

**11.** Composition according to Claim 8 or 9, characterized in that it also contains one or more products selected from nonionic surfactants other than those of formula (X), ionic surfactants, natural and synthetic polymers, mineral, animal or vegetable oils or waxes, polysiloxane derivatives, solvents, propellants, thickeners, preservatives, sunscreens, colourings, fragrances, coemulsifying agents, sterols, moisturizing products and active products for the treatment of skin or scalp conditions.

**12.** Composition according to any one of Claims 8 to 11, characterized in that it takes the form of an aqueous solution, aqueous-alcoholic lotion, oil-in-water or water-in-oil emulsion, microemulsion, microdispersion of lipid vesicles containing or not containing active products, gel or bar or of products for spraying.